# EUROPÄISCHE PATENTSCHRIFT

(11) **EP 0 687 736 B1**
(45) Veröffentlichungstag und Bekanntmachung des Hinweises auf die Patenterteilung: **07.05.1997**
(21) Anmeldenummer: 95108695.8
(22) Anmeldetag: 07.06.1995
(51) Int. Cl.: C12P 41/00, C12P 17/10

(54) **Verfahren zur Herstellung von enantiomerenreinen Lactamen**
Method for the preparation of enantiomerically pure lactams
Méthode pour la préparation de lactomes énantiomériquement purs

(30) Priorität: 15.06.1994 DE 4420751
(43) Veröffentlichungstag der Anmeldung: 20.12.1995
(73) Patentinhaber: BASF AKTIENGESELLSCHAFT, 67056 Ludwigshafen (DE)
(72) Erfinder: Staudenmaier, Horst Ralf, Dr., D-67134 Birkenheide (DE); Hauer, Bernhard, Dr., 67136 Fussgönheim (DE); Balkenhohl, Friedhelm, Dr., D-67117 Limburgerhof (DE); Ladner, Wolfgang, Dr., D-67136 Fussgönheim (DE); Schnell, Ursula, Dr., D-33175 Bad Lippspringe (DE); Pressler, Uwe, Dr., D-67165 Waldsee (DE)

(56) Entgegenhaltungen:
- EP-A- 0 424 064
- EP-A- 0 552 041
- EP-B- 0 059 536
- EP-B- 0 178 826
- EP-B- 0 182 024
- EP-B- 0 384 766
- FR-A- 1 542 706
- PLANT AND CELL PHYSIOLOGY, Bd. 18, Nr. 5, Oktober 1977 Seiten 1173-1176, TAKASHI FUKUMURA 'Partial purification and some properties of an L-alpha-amino-epsilon-caprolactam-hydrolyz ing enzyme from Cryptococcus laurentii'

## Beschreibung

Die vorliegende Erfindung betrifft ein Verfahren zur Herstellung von enantiomerenreinen Lactamen der Formel I 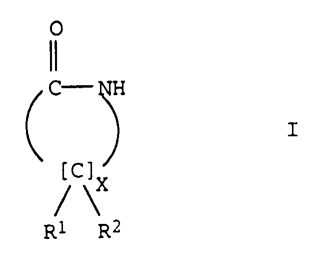 wobei
R¹ und R² unabhängig voneinander H, einen gegebenenfalls substituierten C₁-C₄-Alkylrest, einen gegebenenfalls substituierten C₂-C₄-Alkenylrest, einen gegebenenfalls substituierten Arylrest, (CH₂)ₙ-COOH mit n = 0, 1, 2, 3, und X die Zahl 1, 2, 3, 4, 5 bedeuten.

In EP 424 064 wird die stereoselektive Spaltung von Lactamen mit Hilfe von Mikroorganismen beschrieben. Bei diesem Verfahren werden zwei verschiedene Mikroorganismen verwendet, die jeweils ein Enantiomeres zur entsprechenden Aminosäure hydrolysieren. Dieses Verfahren hat jedoch den Nachteil, daß es auf bestimmte bicyclische Lactame als Substrat beschränkt ist und deshalb nicht breit angewendet werden kann.

Aus DE 21 57 171 ist außerdem ein Verfahren bekannt, mit dem aus racemischem α-Amino-ε-Caprolactam optisch reines Lysin hergestellt werden kann. Das Substratspektrum der hier verwendeten Stämme bzw. des aus diesen Stämmen isolierten Enzyms L-α-Amino-ε-Caprolactam-Hydrolase ist jedoch äußerst eng. So wird dort ausdrücklich festgestellt, daß selbst strukturell sehr ähnliche Verbindungen wie ε-Caprolactam, δ-Valerolactam, α-Butyrolactam, cyclische Oligomere der ε-Aminocapronsäure oder D- und L-Pyrrolidoncarbonsäure nicht hydrolysiert werden (DE 21 57 171, Seite 18, Substratspezifität). Die einzige Ausnahme von dieser Regel ist die Umsetzung von α-Amino-substituierten δ-Valerolactamen, wie sie in EP 357 029 und von T. Fukumura in Plant & Cell Physiol. 18: 1173-1176 (1977) beschrieben sind. Deshalb eignet sich auch dieses Verfahren nicht für eine breite Anwendung, sondern kann ausschließlich für die genannten Reaktionen verwendet werden.

Es bestand daher die Aufgabe, ein Verfahren zur Herstellung von enantiomerenreinen Lactamen bereitzustellen, das die oben beschriebenen strukturellen Beschränkungen nicht aufweist und mit dem sich enantiomerenreine Lactame der Formel I in guter Ausbeute herstellen lassen.

Gefunden wurde ein Verfahren zur Herstellung von enantiomerenreinen Lactamen der Formel I 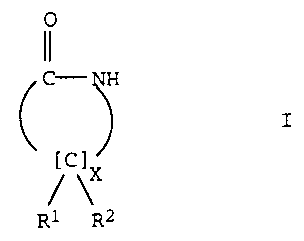 wobei
R¹ und R² unabhängig voneinander H, einen gegebenenfalls substituierten C₁-C₄-Alkylrest, einen gegebenenfalls substituierten C₂-C₄-Alkenylrest, einen gegebenenfalls substituierten Arylrest, (CH₂)ₙ-COOH mit n = 0, 1, 2, 3, und X die Zahl 1, 2, 3, 4, 5 bedeuten,
aus einem Racemat der Formel I, indem man einen Biokatalysator, der selektiv ein Enantiomer von I umsetzt, auf das racemische Gemisch von I einwirken läßt und aus dem entstandenen Produktgemisch das nicht-umgesetzte Enantiomer isoliert.

Für das erfindungsgemäße Verfahren werden von den racemischen Lactamen der Formel I bevorzugt solche eingesetzt, die eine Ringgröße von 4, 5 oder 6 C-Atomen (X = 2, 3 oder 4) besitzen und bei denen alle Substituenten R¹ und R² außer einem die Bedeutung H besitzen, während der eine Substituent eine der oben angegebenen Bedeutungen besitzt.

Besonders bevorzugt unter diesen Lactamen sind die, bei denen der einzige von H verschiedene Substituent R¹ = Methyl oder Vinyl ist.

Für das erfindungsgemäße Verfahren sind als Biokatalysatoren Mikroorganismen geeignet, die die Eigenschaft haben, nur ein Enantiomer der durch Formel I bezeichneten Verbindungen umzusetzen, während sie das andere Enantiomer unbeeinflußt lassen. Solche Mikroorganismen lassen sich leicht nach gängigen Verfahren beispielsweise aus Bodenproben isolieren, wobei eines dieser Verfahren in Beispiel 1 beschrieben ist.

Bevorzugte Mikroorganismen sind Pilze und Bakterien. Besonders bevorzugt sind solche der Gattungen Pseudomonas und Rhodococcus.

Als repräsentative Vertreter for Organismen, die das eine oder das andere Enantiomere selektiv hydrolysieren, wurden folgende Stämme bei Deutsche Sammlung für Mikroorganismen und Zellkulturen GmbH, DSM, am 28.02.94 hinterlegt:
Lu 8676 (Rhodococcus erythropolis): DSM 9002 und
Lu 8745 (Pseudomonas aeruginosa): DSM 9001.

Die Enantioselektivität dieser hinterlegten Stämme ist in den folgenden Beispielen näher bezeichnet.

Ein weiterer geeigneter Mikroorganismus ist Lu 8744, der als Pseudomonas aeruginosa klassifiziert wurde.

Für die stereoselektive Spaltung eines Lactams ist prinzipiell nur ein Enzym notwendig, das das eine Enantiomere zur entsprechenden Aminosäure spaltet, während er das andere Enantiomere unbeeinflußt läßt. Aus diesem Grund können als Biokatalysator für das erfindungsgemäße Verfahren außer ganzen Mikroorganismen auch enzymatische Extrakte daraus oder isolierte Enzyme verwendet werden.

Die Biokatalysatoren können als solche oder in immobilisierter Form, z.B. trägerfixiert, eingesetzt werden.

Nachdem mittels Biokatalysator selektiv ein Enantiomer des Racemats hydrolysiert worden ist, besteht das Produktgemisch aus einem Enantiomer der Aminosäure und einem Enantiomer des Lactams.

Solch ein Produktgemisch läßt sich leicht durch übliche Verfahren aufgrund der unterschiedlichen physiko-chemischen Parameter der Komponenten auftrennen.

Geeignet hierfür sind beispielsweise Destillation, Extraktion, Kristallisation oder chromatographische Verfahren wie Ionenaustauschchromatographie.

Vielfach ist auch nur noch das Enantiomer des nicht umgesetzten Lactams nachweisbar, das andere Enantiomer wird offenbar weiter abgebaut.

Die Umsetzung des Biokatalysators mit dem Racemat geschieht in der Regel bei Temperaturen zwischen 0 und 50, bevorzugt zwischen 20 unc 40°C.

Falls ganze Mikroorganismen als Biokatalysatoren eingesetzt werden, arbeitet man bevorzugt in einem Nährmedium, dem die umzusetzenden Lactame zugegeben werden.

Die Konzentration der Lactame in der Nährlösung beträgt in der Regel 1 bis 100 g Lactam pro Liter Nährlösung.

Werden als Biokatalysatoren Enzymextrakte oder gereinigte Enzyme aus den entsprechenden Mikroorganismen eingesetzt, empfiehlt sich die Umsetzung der Racemate in wäßriger Lösung oder in organischen Lösungsmitteln oder Gemischen aus beiden.

Die Umsetzung führt man vorteilhaft in einem pH-Milieu aus, in dem die Biokatalysatoren noch eine hohe Aktivität aufweisen. Dies ist in der Regel bei pH-Werten zwischen 3 und 9, bevorzugt zwischen 4 und 8 der Fall.

Das erfindungsgemäße Verfahren eignet sich besonders zur Herstellung von enantiomerenreinen substituierten 2-Pyrrolidinonen, etwa methyl- oder vinyl-substituierten 2-Pyrrolidinonen.

Das erfindungsgemäße Verfahren ist hinsichtlich struktureller Parameter der umzusetzenden Lactame wie Ringgröße oder Art und Position des Substituenten wenig eingeschränkt.

Ein weiterer Gegenstand der Erfindung ist, dasjenige Enantiomer, das durch den Biokatalysator umgesetzt wird, zu isolieren und als solches, bzw. nach Wiederüberführen in das Lactam, zu verwenden.

Wird beispielsweise die (S)-Form eines Lactams durch den Biokatalysator selektiv hydrolysiert, so bleibt die (R)-Form des Lactams unverändert zurück und kann direkt aus dem Gemisch isoliert werden.

Die hydrolysierte (S)-Form, die dann als entsprechende enantiomerenreine Aminosäure vorliegt, kann als solche oder nach Zyklisierung zum (S)-Lactam verwendet werden.

Die enantiomerenreine Lactame sind wertvolle Zwischenprodukte für Wirkstoffe auf dem Pharma- und Pflanzenschutzgebiet. Die Erfindung wird durch die folgenden Beispiele weiter veranschaulicht.

### Beispiel 1

Isolierung von Stämmen mit stereoselektiver Lactamaseaktivität aus Bodenproben

Je ca. 2 g einer Bodenprobe wurden in einen Erlenmeyerkolben mit 30 ml Lactamasemedium mit 2 g/l eines Lactams, z.B. Pyrrolidon gegeben und 2 bis 7 Tage bei 25°C schüttelnd inkubiert.

| Lactamasemedium: | |
|---|---|
| MgSO₄ x 7H₂O | 0,5 g/l |
| NaCl | 0,05 g/l |
| CaCl₂ | 0,02 g/l |
| Spurenelementlösung | 2 ml/l |
| KH₂PO₄ | 1,5 g/l |
| K₂HPO₄ | 3,6 g/l |
| Glycerin | 5 g/l |

| Spurenelementlösung | |
|---|---|
| 200 mg/l | Eisen(II)sulfat-1-hydrat |
| 10 mg/l | Zink(II)sulfat-4-hydrat |
| 3 mg/l | Manganchlorid-4-hydrat |
| 30 mg/l | Borsäure |
| 20 mg/l | Cobalt(II)-chlorid-6-hydrat |
| 1 mg/l | Kupfer(II)chlorid-2-hydrat |
| 2 mg/l | Nickel(II)chlorid-6-hydrat |
| 3 mg/l | Natriummolybdat-2-hydrat |
| 500 mg/l | Ethylendiamintetraessigsäure (EDTA) |

Zur Anreicherung geeigneter Stämme wurde je 1 ml dieser Kulturen in neues Medium überimpft, wieder einige Tage geschüttelt und der gleiche Vorgang nochmals wiederholt. Dann wurden die Kulturen auf Agarplatten mit der gleichen Zusammensetzung wie das Anreicherungsmedium ausplattiert. Von diesen Platten wurden Einzelkolonien abgeimpft und getestet:

Die Stämme wurden 2 bis 7 Tage in Lactamasemedium mit 5 g/l des racemischen Lactams 5-Vinylpyrrolidinon gezüchtet. Es wurde täglich eine Probe entnommen und geprüft, ob sich ein optisch aktives Produkt gebildet hatte:
3 ml Kultur wurden mit 3 ml Essigester versetzt, kräftig gemischt und zwecks Phasentrennung zentrifugiert. Nach Filtrieren durch einen 0,2-µm-Filter wurde der Essigesterextrakt direkt für die Drehwertmessung im Polarimeter verwendet.

Aus diesem Screening wurden insgesamt 5 Stämme isoliert, die ein optisch aktives Produkt bildeten. 2 Stämme erzeugten rechtsdrehendes 5-Vinylpyrrolidinon und 3 Stämme erzeugten linksdrehendes 5-Vinylpyrrolidinon.

Analog wurden Stämme in Lactamasemedium mit 5 g/l des racemischen Lactams 3-Methylpyrrolidinon gezüchtet und untersucht. Es wurden 2 Stämme isoliert, die rechtsdrehendes 3-Methylpyrrolidinon erzeugten.

### Beispiel 2

### (S)-5-Vinylpyrrolidinon aus (R,S)-5-Vinylpyrrolidinon

25 ml Lactamasemedium mit 5 g /1 5-Vinylpyrrolidinon wurden mit DSM 9002 angeimpft und 4 Tage bei 30°C geschüttelt. Mit dieser Vorkultur wurden 500 ml des gleichen Mediums als Hauptkultur angeimpft und 5 Tage schüttelnd inkubiert.

Die Zellen wurden abzentrifugiert und der zellfreie Überstand 12 h kontinuierlich mit Essigester extrahiert. Der Essigesterextrakt wurde vom Lösungsmittel befreit und ergab einen Rückstand von 1,43 g 5-Vinylpyrrolidinon. Die Struktur des isolierten Produkts wurde durch NMR bestätigt.

Für das Produkt wurden folgende Drehwerte bestimmt:
[α]_{D} = +27,0 (c = 1, Essigester)
[α]_{D} = +45,3 (c = 1, Ethanol)

Der Vergleich des Drehwerts mit Literaturdaten (GB 21 33 002, Beispiel 10) ergab, daß es sich um das S-Enantiomere handelt.

Die Bestimmung des Enantiomerenverhältnisses wurde mit Hilfe von chiraler GC durchgeführt und ergab einen Enantiomerenüberschuß von 98,6 % ee (Säule: Cyclodex-β-I/P®, 50 m x 0,32 mm).

Das Enantiomer wurde in hoher Ausbeute erhalten. Durch die Essigesterextraktion wurden noch geringe Mengen organischer Bestandteile mitisoliert, die jedoch leicht abtrennbar sind.

### Beispiel 3

### (R)-5-Vinylpyrrolidinon aus (R,S)-5-Vinylpyrrolidinon

500 ml Lactamasemedium mit 2 g/l Pyrrolidon wurde mit DSM 9001 angeimpft und 2 Tage bei 30°C geschüttelt. Dann wurden 2,5 g racemisches 5-Vinylpyrrolidinon zugegeben und die Kultur 5 Tage weitergeschüttelt. Die Kultur wurde abgebrochen und wie in Beispiel 2 aufgearbeitet. Man erhielt einen Rückstand von 0,82 g (R)-5-Vinylpyrrolidinon.

Das Produkt hatte einen Drehwert von [α]_{D} = -27,7 (c = 1, Essigester).

### Beispiel 4

### Stereoselektiver Umsatz von verschiedenen Lactamen und Säureamiden

Die Stämme aus Beispiel 1 wurden 2 bis 7 Tage in Lactamasemedium mit 5 g/l einer der in Tabelle 1 aufgelisteten Verbindungen gezüchtet. Aus den Kulturen wurden im Abstand von 1 bis 2 Tagen Proben entnommen und geprüft, ob sich ein optisch aktives Produkt gebildet hatte. Die Gewinnung der Proben erfolgte wie in Beispiel 1.

**Tabelle**

| Substrat | Stamm | |
|---|---|---|
| | Produkt linksdrehend | Produkt rechtsdrehend |
| 5-Vinylpyrrolidinon | Lu 8744, 8745, 8746 | Lu 8676, 8743 |
| 3-Methylpyrrolidinon | Lu 8744, 8746 | Lu 8747, 8748 |
| 6-Phenylvalerolactam | Lu 8744, 8745, 8746 | |

## Patentansprüche

1. Verfahren zur Herstellung von enantiomerenreinen Lactamen der Formel I 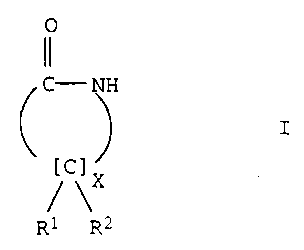 wobei
R¹ und R² unabhängig voneinander H, einen gegebenenfalls substituierten C₁-C₄-Alkylrest, einen gegebenenfalls substituierten C₂-C₄-Alkenylrest, einen gegebenenfalls substituierten Arylrest, (CH₂)ₙ-COOH mit n = 0, 1, 2, 3, und X die Zahl 1, 2, 3, 4, 5 bedeuten,
aus einem Racemat der Formel I, indem man einen Biokatalysator, der selektiv ein Enantiomer von I umsetzt, auf das racemische Gemisch von I einwirken läßt und aus dem entstandenen Produktgemisch das nicht-umgesetzte Enantiomer isoliert.

2. Verfahren nach Anspruch 1, dadurch gekennzeichnet, daß als Biokatalysator ein Mikroorganismus der Gattung Rhodococcus oder Pseudomonas verwendet wird.

3. Verfahren nach Anspruch 1 oder 2, dadurch gekennzeichnet, daß das (S)-Enantiomer von I mittels DSM 9002 als Biokatalysator hergestellt wird.

4. Verfahren nach Anspruch 1 oder 2, dadurch gekennzeichnet, daß das (R)-Enantiomer von I mittels DSM 9001 als Biokatalysator hergestellt wird.

5. Verfahren nach Anspruch 3, dadurch gekennzeichnet, daß (S)-5-Vinylpyrrolidinon hergestellt wird.

6. Verfahren nach Anspruch 4, dadurch gekennzeichnet, daß (R)-5-Vinylpyrrolidinon hergestellt wird.

## Claims

1. A process for preparing enantiomerically pure lactams of the formula I 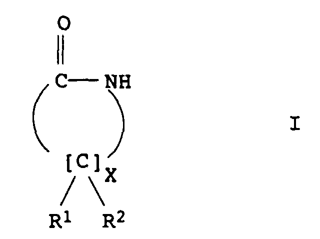 where
R¹ and R² are each, independently of one another, H, unsubstituted or substituted C₁-C₄-alkyl, unsubstituted or substituted C₂-C₄-alkenyl, unsubstituted or substituted aryl, (CH₂)ₘ-COOH with n = 0, 1, 2, 3, and X is 1, 2, 3, 4, 5,
from a racemate of the formula I by allowing a biocatalyst which selectively converts one enantiomer of I to act on the racemic mixture of I, and isolating the unconverted enantiomer from the resulting mixture of products.

2. A process as claimed in claim 1, wherein a microorganism of the genus Rhodococcus or Pseudomonas is used as biocatalyst.

3. A process as claimed in claim 1 or 2, wherein the (S) enantiomer of I is prepared using DSM 9002 as biocatalyst.

4. A process as claimed in claim 1 or 2, wherein the (R) enantiomer of I is prepared using DSM 9001 as biocatalyst.

5. A process as claimed in claim 3, wherein (S)-5-vinylpyrrolidinone is prepared.

6. A process as claimed in claim 4, wherein (R)-5-vinylpyrrolidinone is prepared.

## Revendications

1. Procédé de préparation de lactames énantiomériques purs de la formule I : 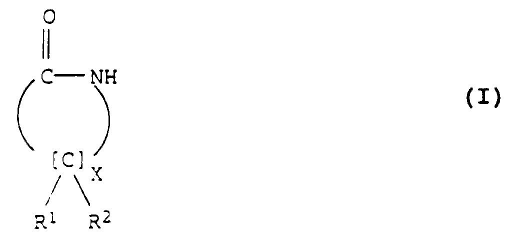 dans laquelle
R¹ et R² représentent chacun indépendamment un atome d'hydrogène, un radical alkyle en C₁ à C₄ éventuellement substitué, un radical alcényle en C₂ à C₄ éventuellement substitué, un radical aryle éventuellement substitué, un radical (CH₂)ₙ-COOH avec n = 0, 1, 2, 3 et X représente le nombre 1, 2, 3, 4 ou 5,
à partir d'un racémique de la formule I, pour autant que l'on fasse agir un biocatalyseur qui convertit sélectivement un énantiomère de I sur le mélange racémique de I et on isole l'énantiomère non converti à partir du mélange de produits formé.

2. Procédé suivant la revendication 1, caractérisé en ce que l'on utilise, à titre de biocatalyseur, un micro-organisme du genre Rhodococcus ou Pseudomonas.

3. Procédé suivant la revendication 1 ou 2, caractérisé en ce que l'on prépare l'énantiomère (S) à partir de I avec DSM 9002 à titre de biocatalyseur.

4. Procédé suivant la revendication 1 ou 2, caractérisé en ce que l'on prépare l'énantiomère (R) à partir de I avec DSM 9001 à titre de biocatalyseur.

5. Procédé suivant la revendication 3, caractérisé en ce que l'on prépare la (S)-5-vinylpyrrolidinone.

6. Procédé suivant la revendication 4, caractérisé en ce que l'on prépare la (R)-5-vinylpyrrolidinone.
